Europäisches Patentamt

European Patent Office

Office européen des brevets

(11) Numéro de publication: **0 326 494 B1**

(19)

(12) **FASCICULE DE BREVET EUROPEEN**

(45) Date de publication de fascicule du brevet: **22.09.93** �351�357 Int. Cl.⁵: **G01N 31/12**

(21) Numéro de dépôt: **89400233.6**

(22) Date de dépôt: **27.01.89**

(54) **Dispositif de mesurage de la puissance calorifique véhiculée par un courant de matière combustible.**

(30) Priorité: **29.01.88 FR 8801086**

(43) Date de publication de la demande:
**02.08.89 Bulletin 89/31**

(45) Mention de la délivrance du brevet:
**22.09.93 Bulletin 93/38**

(84) Etats contractants désignés:
**AT BE CH DE ES GB GR IT LI LU NL SE**

(56) Documents cités:
**EP-A- 0 060 681       EP-A- 0 098 716**
**EP-A- 0 224 655       FR-A- 2 564 591**
**GB-A- 1 056 540       GB-A- 2 099 589**

(73) Titulaire: **GAZ DE FRANCE (SERVICE NATIO-NAL)**
**23 rue Philibert-Delorme**
**F-75017 Paris(FR)**

(72) Inventeur: **Guillet, Rémi**
**46 rue Albert Francon**
**F-93380 Pierrefitte(FR)**

(74) Mandataire: **Thévenet, Jean-Bruno et al**
**Cabinet Beau de Loménie 158, rue de l'Université**
**F-75340 Paris Cédex 07 (FR)**

EP 0 326 494 B1

# Description

La présente invention a pour objet un dispositif de mesurage de la puissance calorifique véhiculée par un courant, notamment gazeux, de matière énergétique combustible circulant dans une conduite principale.

Selon l'état de la technique, le mesurage de la puissance calorifique fait toujours appel à deux grandeurs "primaires", à savoir :
- le débit massique de la matière énergétique,
- le pouvoir calorifique de cette matière.

Si le débit massique est une mesure accessible au plus grand nombre des techniciens, avec une incertitude elle même appréhendable et liée directement aux moyens mis en oeuvre, pouvant être opérée en continu dans la plupart des cas, le pouvoir calorifique, lui, reste une grandeur délicate à définir, faisant appel à des moyens relativement lourds (investissements souvent importants, techniciens confirmés,...) s'exprimant par valeurs discrètes (par exemple il faut environ 15 minutes d'analyse chromatographique sur un échantillon prélevé pour établir son Pouvoir Calorifique).

La difficulté de mesurer de façon commode le pouvoir calorifique d'une matière énergétique a pour conséquence directe et inconvénient majeur le fait que l'énergie est le plus souvent vendue "au poids".

Dans le cas des combustibles gazeux, notamment ceux distribués par réseaux, ceci se traduit par le fait que le distributeur achète à son fournisseur (le transporteur) et vend à son client le gaz en se basant sur :
- le volume normal, ou volume exprimé dans les conditions de référence dites normales, du gaz effectivement mesuré,
- une estimation du pouvoir calorifique.

Bien entendu, ceci fait l'objet de controverses, voire de litiges, lorsque le pouvoir calorifique du gaz distribué est susceptible de grandes variations. A titre d'exemple, en France, la fourchette de variation réglementairement possible représente approximativement 18 % de la valeur moyenne du gaz naturel distribué par le réseau de gaz dit "H".

On connaît par le document GB-A-1 056 540 un procédé et un dispositif qui assurent le prélèvement de produits de combustion dans une chaudière, puis la combustion complète, dans une chambre de combustion annexe, de la partie du combustible imbrûlé présente dans les produits de combustion prélevés , la détermination du facteur d'air s'appliquant à cette combustion annexe et la déduction de la puissance calorifique à partir de la mesure du facteur d'air et de la mesure du débit massique d'air comburant nécessaire à la combustion dans la chaudière.

Le document GB-A-2 099 589 décrit un procédé de détermination du pouvoir calorifique d'un courant gazeux circulent dans une conduite, selon lequel un échantillon du courant gazeux est prélevé sur la ligne puis mélangée à de l'air pour être appliqué à une cellule de mesure dans laquelle on mesure la quantité d'oxygène nécessaire à la combustion de l'échantillon prélevé et on en déduit la valeur du pouvoir calorifique du courant gazeux.

La présente invention vise à remédier aux inconvénients précités et à permettre de mesurer effectivement avec précision la puissance calorifique transmise par un courant, notamment gazeux, de matièr énergétique, en réalisent des mesures en continu à partir de matériels simples et sans qu'une intervention humaine soit nécessaire pour la conduite des mesures en continu.

Ces buts sont atteints grâce à un dispositif de mesure de la puissance calorifique véhiculée par un courant notamment gezeux de matière énergétique combustible circulent dans une conduite principale de transport ou de distribution en transit d'un courant gazeux de matière combustible vers une zone aval d'utilisation à pression variable comprenant une chambre de combustion propre au dispositif de mesure pour provoquer la combustion d'une faible fraction connue du débit massique de la matière combustible circulent dans le conduite,et une chaîne de mesure du facteur d'air f appliqué à ladite chambre de combustion ou de régulation de la combustion dans ladite chambre de combustion pour maintenir le facteur d'air f constant, caractérisé en ce qu'il comprend une tuyère principale à col sonique à section variable disposée dans la conduite principale, et un circuit de dérivation d'une faible fraction connue de débit massique du courant gazeux circulant dans la conduite principale, pour appliquer ladite faible fraction à ladite chambre de combustion contrôlée, en ce que le circuit de dérivation prend naissance dans une zone de la conduite principale située en amont de la tuyère principale et comporte une tuyère secondaire à col sonique de géométrie fixe, en ce que la chambre de combustion du circuit de dérivation est alimentée en air comburant par une canalisation sur laquelle est monté un dispositif de comptage du débit massique d'air comburant utilisé pour la combustion de ladite faible fraction connue et appliqué à ladite chambre de combustion et en ce qu'il comprend des moyens de calcul et d'affichage ou d'enregistrement reliés audit dispositif de comptage et à ladite chaîne de mesure ou de régulation du facteur d'air f pour déterminer et afficher ou enregistrer la valeur de la puissance calorifique Pu de la matière circulant dans ladite conduite principale à partir des informations de débit massique d'air comburant délivrées par ledit dispositif de comptage et de facteur d'air f délivrées par ladite chaîne de mesure ou de régulation.

La tuyère principale à col sonique qui assure une fonction de régulation de pression aval et présente un col dont la section de passage est variable, peut être définie par la position d'une ogive mobile disposée à l'intérieur du corps de la tuyère et déplaçable en translation selon l'axe de la tuyère par rapport audit col, et un capteur de position est utilisé pour détecter les déplacements et la position de l'ogive mobile et fournir aux moyens de calcul et d'affichage une information concernant la position longitudinale de cette ogive.

Dans ce cas, seul est constant le débit de matière énergétique dans le circuit dérivé. Toutefois, le repérage de l'emplacement de l'ogive permet de connaître le valeur du coefficient K représentant le quotient du débit du circuit dérivé par rapport au débit du circuit principal.

Avantageusement, le circuit de dérivation présente des portions de section réduite créent une localisation des pertes de charges.

Dans ce cas, de préférence, la conduite principale est munie d'un filtre disposé en amont de la zone de raccordement du circuit de dérivetion à la conduite principale. Le circuit de dérivation peut en outre être muni d'un second filtre sans pertes de charge significatives disposé en amont de la zone de localisation des pertes de charge.

Selon une caractéristique particulière avantageuse le chaîne de mesure du facteur d'air f comprend des moyens d'analyse de le teneur en oxygène des produits de combustion issus de le chambre de combustion, le dispositif de comptage du débit massique d'air comburant comprend également des moyens d'analyse de la teneur en oxygène de l'air comburant et lesdits moyens d'analyse de teneur en oxygène sont constitués par un analyseur unique de taux d'oxygène appliqué alternativement aux conduits d'évacuation des produits de combustion et à la canalisation d'alimentation en air comburant.

D'autres caractéristiques et avantages de l'invention ressortiront de le description suivante de modes particuliers de réalisation de l'invention, donnés à titre d'exemples non limitatifs, en référence aux dessins annexés sur lesquels :

- la figure unique est une vue schématique d'un mode pardiculier de réalisation de l'invention appliqué à une zone de transit d'une conduite principale de transport ou de distribution d'un combustible gazeux et selon lequel des mesures de puissance calorifique sont effectuées à l'aide d'un circuit dérivé spécifique conformément à l'invention, une tuyère sonique à col de section variable étant mise en oeuvre dans la conduite principale de transport ou de distribution.

On rappellera tout d'abord que pour un combustible donné, et un air comburant défini par sa teneur en oxygène, il y a bien sûr correspondance biunivoque entre le pouvoir calorifique $P_c$ et le volume de l'air théorique $V_A$ nécessaire à la combustion stoechiométrique de l'unité de masse selon la formule suivante :

$$V_A/P_C = C \qquad (1)$$

où C est une constante.

Par ailleurs, il est à observer que ce rapport $V_A/P_C$ varie peu avec la nature du combustible, notamment dans le cas des gaz naturels.

A titre d'exemple, les gaz naturels susceptibles de transiter par le réseau de distribution de Gaz de France présentent un rapport $V_A/P_C$ stable à 2 pour mille près.

Le présente invention prend en compte la stabilité de ce rapport et permet de réaliser des mesures de puissance calorifique de façon plus simple que selon les procédés connus.

Pour cela, conformément au procédé selon l'invention, on met en oeuvre une combustion complète définie par son taux d'aération ou facteur d'air f, f étant défini comme le rapport du volume d'air effectivement utilisé va eu volume d'air $V_A$ nécessaire à le combustion stoechiométrique : $f = va/V_A$ (2). Dans ce cas, le comptage de le puissance calorifique Pu peut être issu de la seule mesure de débit massique d'air comburant.

On rappelle que la puissance calorifique Pu est définie de le façon suivante :

$$Pu = Qc \cdot Pc \qquad (3)$$

où Qc est le débit massique du combustible et Pc est le pouvoir calorifique.

Il ressort de le combinaison des équations (1) à (3) que

$$Pu = Qc \, va/(f.C) \qquad 4)$$

Or Qc.va représente le débit massique d'air $Q_{air}$ effectivement utilisé ou air comburant associé au débit Qc, de sorte que l'équation (5) devient :

$$Pu = Q_{Air}. \, 1/(f.C) \qquad (5)$$

Dans la mesure où le facteur d'air f est maintenu constant par une régulation, la mesure du débit massique d'air comburant Qair permet de déduire directement, par un simple coefficient de proportionnalité, la valeur de la puissance calorifique.

On notera alors que la valeur de f peut être choisie quelconque, bien qu'il soit avantageux de choisir une valeur du facteur d'air f égale à 1 ou voisine de 1.

Dans le cas où le facteur d'air f ne fait pas l'objet d'une régulation, celui-ci se trouve susceptible de varier notamment à l'inverse des variations du pouvoir comburivore VA du combustible considéré.

Pour déterminer le valeur de le puissance calorifique, il est alors nécessaire de mesurer le débit massique d'air $q_{air}$, et le facteur d'air f, par exemple à l'aide d'une analyse de la teneur en oxygène résiduel dans les produits de la combustion.

La précision obtenue sur la valeur de la puissance calorifique Pu est celle observée sur la mesure du débit univoque d'air QA augmentée d'un facteur de l'ordre de 3 à 7 pour mille pour un gaz tel que le gaz "H" distribué par Gaz de France. Le facteur de 7 pour mille tient compte de la précision attendue dans le cas la régulation du facteur d'air.

Selon l'invention, on réalise ainsi des mesures de puissance calorifique avec une précision qui ne dépend que de celle d'un dispositif de comptage de débit d'air, à laquelle on ajoute une incertitude de seulement quelques pour mille.

Au contraire, selon les procédés classiques imposant la mesure d'un débit massique de combustible ainsi qu'une mesure de pouvoir calorifique, si la précision sur la mesure du débit massique de combustible peut être de l'ordre de grandeur de celle d'une mesure de débit messique d'air, une incertitude sur l'évaluation du pouvoir calorifique de quelques pour mille, nécessite des moyens de mesure le plus souvent dissuasifs. Le procédé selon l'invention permet donc de généraliser les mesures de puissance calorifique avec une précision comparable aux meilleurs procédés de laboratoire, tout en autorisant des mesures en continu.

Un mode particulier de réalisation de l'invention sera maintenant décrit de façon plus détaillée en référence à la figure unique.

On a représenté sur le figure un mode particulier de réalisation dans lequel un comptage de débit massique d'air en vue de déterminer une puissance calorifique est effectué sur une conduite principale 1 de transport en transit d'un courant gazeux $Q_{gaz}$ de matière combustible, qui peut être par exemple du gaz naturel, vers une zone aval d'utilisation à pression P2 variable. Le mode de réalisation de la figure peut ainsi être appliqué à un poste de livraison de fluide combustible du transporteur au distributeur ou du distributeur à son client et permet de déterminer la puissance calorifique d'un fluide gazeux. Dans ce cas, le dispositif de mesure comprend un circuit dérivé 101 afin de prélever un débit de gaz $q_{gaz}$ très inférieur au débit initial $Q_{gaz}$.

Le circuit dérivé 101 permet de prélever en une zone 8 une fraction $q_{gaz}$ constante et très faible du débit principal de fluide combustible $Q_{gaz}$ afin d'alimenter à travers des canalisations de géométrie fixe une chambre de combustion annexe 110. Une chaîne 104 de régulation du facteur d'air ou 104' de mesure du facteur d'air est associée à la chambre de combustion annexe 110.

La chaîne de régulation de facteur d'air 104 comprend une sonde 141 et un organe 140 de modulation de la quantité d'air comburant appliquée à la chambre de combustion 110 par la canalisetion 103 débouchant dans une zone 130 voisine de l'extrémité de la canalisetion dérivée 101.

Le capteur 141, utilisé dans le cas où le facteur d'air est régulé, peut être constitué par exemple par une sonde à détection de seuil, qui peut être avantageusement une sonde au zirconium, et qui peut présenter une précision de l'ordre de 3 à 5 pour mille.

Comme indiqué plus haut, la chaîne 104 de régulation de facteur d'air n'est pas absolument indispensable. Si la chaîne 104 de régulation n'existe pas, celle-ci est remplacée par une chaîne de mesure 104' (représentée en traits mixtes sur la figure) qui transmet à un dispositif de calcul 160 la valeur du facteur d'air f obtenue après analyse de l'oxygène résiduel dans les produits de la combustion complète, à l'aide d'un capteur 141 qui n'est alors plus à détection de seuil mais présente une plage de mesure étendue.

Un compteur de débit d'air 106 sert à la mesure du débit massique d'air comburant $q_{air}$ introduit par la canalisation 103 et fournit au dispositif de calcul 160 une valeur mesurée du débit massique d'air comburant utilisé pour la combustion de la fraction $q_{gaz}$ de fluide combustible appliquée par la canalisation 101 à la chambre de combustion annexe 110 propre au dispositif de mesure. Le dispositif 160 de calcul et d'affichage ou d'enregistrement de la puissance celorifique reçoit en entrée des informations du compteur de débit d'air 106 et le cas échéant de la chaîne 104' d'analyse du facteur d'air f.

Le dispositif 160 peut comprendre en outre des circuits d'intégration dans le temps des valeurs de puissance calorifique calculées pour délivrer en sortie des valeurs de quantité d'énergie.

Le dispositif 106 de mesure de débit massique d'air comprend avantageusement pour plus de précision des capteurs de mesure de température, de pression, d'hygrométrie et le cas échéant de teneur en oxygène. La connaissance de ces divers paramètres permet de déterminer avec précision en fonction du volume d'air ayant parcouru le compteur 106 par unité de temps le débit massique d'air.

Dans la présente description on considère que l'on applique par la canalisation 103 un débit d'air comburant dont la valeur est déterminée par le dispositif de mesure 106, mais l'invention prend naturellement en compte également le cas où l'on appliquerait par la canalisation 103 de l'oxygène pur.

Compte tenu du fait que la pression dans la zone 8 de la conduite principale 1 où prend naissance le canalisation de dérivation 101 est plus élevée que la pression dans la chambre de combustion annexe 110, une zone Z3 de la canalisation de dérivation 101 correspond de préférence à une zone de pertes de charge localisées du circuit dérivé, ce qui peut permettre un étalonnage en laboratoire du circuit dérivé.

La chambre de combustion contrôlée annexe 110 peut être réalisée de diverses manières et peut notamment être du type à combustion catalytique.

Le dispositif 106 de mesure de débit massique d'air comburant peut comprendre divers composants qui sont eux mêmes classiques. Il est ainsi possible d'utiliser une pompe volumétrique basse pression, par exemple à crémeillère, pour produire et mesurer le débit de fluide gazeux.

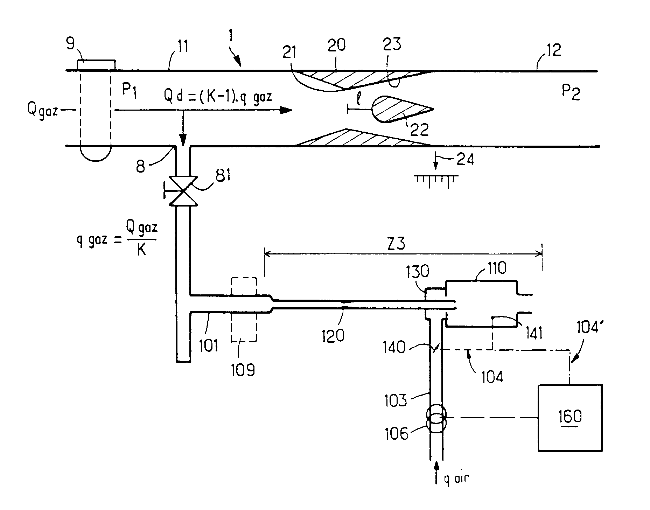

Dans le cas d'une conduite principale 1 de transport ou de distribution d'un fluide gazeux correspondant à une zone de transit, la géométrie ou les coefficients de débit du réseau aval varient constamment en fonction notamment du positionnement de divers éléments de robinetterie gérés par les utilisateurs et clients. Dans ce cas, le coefficient K correspondant au rapport entre le débit principal de gaz $Q_{gaz}$ dans la conduite principale 1 et la fraction de débit de gaz $q_{gaz}$ prélevée dans la cenalisation de dérivation est variable. Le mode de réalisation de la figure 3 permet de remédier à ce problème et présente diverses dispositions particulières qui permettent de s'affranchir des variations de la pression aval P2 et par suite de maintenir un rapport constant entre le débit gazeux $q_{gaz}$ prélevé dans le circuit 101 de dérivation et le débit principal $Q_{gaz}$ circulent dans la conduite principale 1.

Dans le mode de réalisation de le figure, une tuyère sonique 20 a été disposée dans la conduite principale 1 pour définir un tronçon de conduite amont 11 et un tronçon de conduite aval 12. La tuyère sonique convergente divergente 20 comporte un col 21 de section fixe et détermine un débit de gaz Qd dans la conduite principale 1 qui est indépendant de le pression aval P2 et ne dépend que de la pression amont P1, de la section du col 21 de la tuyère 20 et bien sûr des caractéristiques du fluide (densité, viscosité,...).

Le circuit de dérivation 101 comprend lui-même une tuyère 120 à col sonique et le circuit dérivé 101 est raccordé à la conduite principale 1 immédiatement en amont de la tuyère 20 placée sur le circuit principal, dans une zone 8 du tronçon amont 11 de la conduite principale 1.

Le circuit de dérivation 101 comprend au voisinage de la zone 8 de raccordement à la conduite principale 1 un robinet 81 à passage direct ne donnant pas lieu à des pertes de charge significatives et peut aussi comporter un filtre 109 également à pertes de charge non significatives. Le circuit dérivé 101 est à géométrie fixe et comporte une zone Z3 à localisation des pertes de charge, dans laquelle est disposée la tuyère 120 à col fixe.

Le circuit dérivé 101 débouche dans une chambre de combustion annexe 110 qui peut être par exemple du type à combustion catalytique, à laquelle est associée une chaîne 104 de régulation de facteur d'air ou 104' de mesure du facteur d'air et qui est équipée d'une conduite 103 d'alimentation d'air comburant sur laquelle est disposé un dispositif 106 de mesure de débit massique d'air qair associé à un dispositif de calcul et d'affichage 160. Un filtre 9 sans pertes de charge significatives peut également être disposé sur la conduite principale 1 en amont de la zone 8 dans laquelle prend naissance le circuit de dérivation 101.

Avec le dispositif de mesure représenté sur la figure, il est possible de déterminer une puissance calorifique avec une grande précision à partir de la mesure de débit d'air effectuée par le dispositif de mesure 106, sous réserve de disposer d'une chaîne 104 de régulation ou 104' de mesure de facteur d'air précise et de déterminer également avec précision le rapport des sections de la tuyère principale 20 et de la tuyère secondaire 120. Le rapport de ces sections peut être déterminé lors du montage ou peut être déterminé par étalonnage du circuit principal et du circuit dérivé. La présence d'un robinet 81 permet de réaliser l'étalonnage du circuit dérivé d'une façon commode.

On notera que la mesure du débit d'air $q_{air}$ à l'aide du dispositif 106 porte sur une valeur relativement peu variable dès lors que le raccordement du circuit dérivé 101 est effectué en une zone 8 où la pression P1 dans la conduite principale est relativement stable. Ceci permet un meilleur choix du calibre du compteur de débit massique d'air et donc une précision optimale.

Dans le cas du mode particulier de réalisation représenté sur la figure, le tuyère principale 20 disposée dans le conduite principale 1 est de type sonique mais présente un col 21 à section variable du fait de la présence d'une ogive 22 disposée axialement à l'intérieur du corps 23 de le tuyère 20. Dans ce cas, la tuyère sonique à col variable 20 peut présenter une fonction de régulation de la pression aval P2 et peut également constituer un compteur du débit de fluide combustible gazeux par courent la conduite principale 1.

Dans le mesure où la section du col de le tuyère 20 est variable en fonction de la position de l'ogive mobile 22 selon l'axe de la tuyère, le rapport entre le débit de gaz dans la conduite principale 1 et le débit de gaz dérivé dans le circuit de dérivation 101 est fonction de la position de l'ogive 22. Par suite, le coefficient de proportionnalité entre la valeur du débit d'air comburant mesuré par le dispositif de mesure 106 et la puissance calorifique distribuée dans la conduite principale 1 est lui-même dépendant de la position de l'ogive mobile 22. Le position de l'ogive 22 peut toutefois être déterminée avec précision en permanence par exemple à l'aide d'un organe de repérage 24 solidaire de l'ogive 22. En effectuant un étalonnage préalable, il est facile de déterminer l'évolution du coefficient de proportionnalité et ainsi de déterminer la puissance calorifique distribuée, à partir du débit d'air mesuré, et du repérage de le position l de l'ogive 22 selon l'axe de la tuyère 20.

Le mode de réalisation représenté sur la figure permet ainsi de combiner un dispositif de mesure calorifique avec un régulateur compteur tel que par exemple celui qui est décrit dans le brevet français 2 341 131.

Dans le cas du mode de réalisation décrit, il est bien sûr aussi possible d'utiliser divers dispositifs, par exemple de type pneumatique, pour maintenir constant, ou mesurer en permanence, le coefficient K de répartition du débit $Q_{gaz}$ du fluide combustible entre la branche principale de la conduite 1 et le circuit dérivé 101 prenant naissance dans le zone 8 de la conduite principale 1. En particulier, si l'on dispose d'un dispositif de mesure de comptage du débit massique du gaz $Q_{gaz}$ dans la conduite principale 1, il est possible de déterminer le facteur K de proportionnalité entre le débit $Q_{gaz}$ et le débit dérivé $q_{gaz}$ en effectuant une mesure du débit de gaz dérivé $q_{gaz}$. Cette mesure du débit de gaz dérivé $q_{gaz}$ peut être effectuée de manière préférentielle soit grâce à la mise en oeuvre d'une tuyère à col fixe, notamment si l'on dispose d'un point de raccordement à pression régulée, soit en fin de circuit dérivé 101, immédiatement avant la combustion contrôlée dans la chambre 110, pour bénéficier des avantages du point de vue de la précision, des coûts et d'un comptage dans les conditions proches de l'ambiance.

Dans le cas de l'utilisation d'un circuit dérivé, on procède à une localisation et une répartition des pertes de charge du circuit dérivé pour permettre le cas échéant un étalonnage des deux circuits principal et dérivé pris séparément et un dimensionnement et un fonctionnement optimisés de la tuyère 120 placée dans le circuit dérivé 101.

On notera qu'un circuit dérivé unique 101 pourrait être associé à plusieurs circuits principaux de transport de fluide combustible présentant des calibres différents et donc équipés de tuyères de calibres différents, sous réserve que chacune des conduites principales de celibres différents soient équipées d'un robinet automatique à passage direct n'introduisant pas de perte de charges significatives et permettant un raccordement par alternance aux divers tronçons de conduites de calibres différents. Ceci peut notamment permettre d'accroître la précision des mesures de puissance calorifique en choisissent à chaque fois le calibre le plus approprié.

On notera que dans tous les modes de réalisation qui viennent d'être décrits les débits massiques d'air mesurés par le dispositif de mesure 106 sont mesurés dans des conditions proches des conditions ambiantes ce qui est particulièrement avantageux, du fait notamment qu'il est possible de disposer de nombreux matériels pouvant s'adapter aux diverses conditions de mesure.

## Revendications

1. Dispositif de mesure de la puissance calorifique véhiculée par un courant notamment gazeux de matière énergétique combustible circulant dans une conduite principale (1) de transport ou de distribution en transit d'un courant gazeux de matière combustible vers une zone aval d'utilisation à pression variable, comprenant une chambre de combustion (110) propre au dispositif de mesure pour provoquer la combustion d'une faible fraction ($q_{gaz}$) connue du débit massique ($Q_{gaz}$) de la matière combustible circulant dans la conduite (1), et une chaîne (104,104') de mesure du facteur d'air (f) appliqué à ladite chambre de combustion (110) ou de régulation de la combustion dans ladite chambre de combustion (110) pour maintenir un facteur d'air (f) constant, caractérisé en ce qu'il comprend une tuyère principale (20) à col sonique à section variable disposée dans la conduite principale (1), et un circuit (101) de dérivation d'une faible fraction ($q_{gaz}$) connue de débit massique ($Q_{gaz}$) du courant gazeux circulant dans la conduite principale (1), pour appliquer ladite faible fraction ($q_{gaz}$) à ladite chambre de combustion contrôlée (110), en ce que le circuit (101) de dérivation prend naissance dans une zone (8) de la conduite principale (1) située en amont de la tuyère principale (20) et comporte une tuyère secondaire (120) à col sonique de géométrie fixe, en ce que la chambre de combustion (110) du circuit de dérivation est alimentée en air comburant par une canalisation (103) sur laquelle est monté un dispositif (106) de comptage du débit massique ($q_{air}$) d'air comburant utilisé pour la combustion de la fraction ($q_{gaz}$) et appliqué à ladite chambre de combustion (110) et en ce qu'il comprend des moyens de calcul et d'affichage ou d'enregistrement (160) reliés audit dispositif de comptage (106) et à ladite chaîne (104, 104') de mesure ou de régulation du facteur d'air (f) pour déterminer et afficher ou enregistrer la valeur de la puissance calorifique (Pu) de la matière circulent dans ladite conduite principale (1) à partir des informations de débit massique ($q_{air}$) d'air comburant délivrées par ledit dispositif de comptage (106) et de facteur d'air (f) délivrées par ladite chaîne (104, 104') de mesure ou de régulation.

2. Dispositif selon la revendication 1, caractérisé en ce que la tuyère principale (20) à col sonique assurant une fonction de régulation de pression aval présente un col dont la section de passage variable est définie par la position d'une ogive mobile (22) disposée à l'intérieur du corps de la tuyère (22) et déplaçable en translation selon l'axe de la tuyère par rapport audit col, et en ce qu'un capteur de position (24) est utilisé pour détecter les déplacements et la position de l'ogive mobile (22) et fournir aux moyens de calcul et d'affichage (160) une information concernant la position longitudinale de cette ogive (22).

3. Dispositif selon l'une quelconque des revendications 1 et 2, caractérisé en ce que le circuit de dérivation (101) présente des portions de section réduite créent une localisation des pertes de charges (zone Z2 ou Z3).

4. Dispositif selon l'une quelconque des revendications 1 et 2, caractérisé en ce que la conduite principale (1) est munie d'un filtre (9) disposé en amont de la zone (8) de raccordement du circuit de dérivation (101) à la conduite principale (1).

5. Dispositif selon l'une quelconque des revendications 1 et 2, caractérisé en ce que le circuit de dérivation (101) est muni d'un second filtre (109) sans pertes de charge significatives disposé en amont de la zone (Z3) de localisation des pertes de charge.

6. Dispositif selon l'une quelconque des revendications 1 et 2, caractérisé en ce que le circuit de dérivation (101) est équipé d'un robinet à passage direct (81) disposé à l'entrée dudit circuit (101).

7. Dispositif selon l'une quelconque des revendications 1 et 2, caractérisé en ce que la chambre de combustion contrôlée (110) est du type à combustion catalytique.

8. Dispositif selon l'une quelconque des revendications 1 à 7, caractérisé en ce que le dispositif (106) de comptage de débit massique d'air comprend une pompe volumétrique basse pression.

9. Dispositif selon l'une quelconque des revendications 1 à 8, caractérisé en ce qu'il comprend un dispositif de détection des variations du facteur d'air de combustion comprenant une sonde à seuil pour la mesure de l'oxygène résiduel dans les produits de combustion.

10. Dispositif selon l'une quelconque des revendications 1 à 9, caractérisé en ce que le dispositif (106) de comptage du débit massique d'air comburant comprend des moyens de mesure de volume, de température, de pression et d'hygrométrie de l'air comburant appliqué à la chambre de combustion (110) propre au dispositif.

11. Dispositif selon l'une quelconque des revendications 1 à 8, caractérisé en ce que la chaîne (104') de mesure du facteur d'air (f) comprend des moyens d'analyse de la teneur en oxygène des produits de combustion issus de la chambre de combustion (110) propre au dispositif.

12. Dispositif selon la revendication 11, caractérisé en ce que le dispositif (106) de comptage du débit massique d'air comburant comprend également des moyens d'analyse de la teneur en oxygène de l'air comburant et en ce que lesdits moyens d'analyse de teneur en oxygène sont constitués par un analyseur unique de taux d'oxygène appliqué alternativement aux conduits d'évacuation des produits de combustion et à la canalisation (103) d'alimentation en air comburant.

13. Dispositif selon l'une quelconque des revendications 1 à 12, caractérisé en ce que les moyens de calcul et d'affichage ou d'enregistrement (160) reliés au dispositif de comptage (106) et à la chaîne (104, 104') de mesure ou de régulation du facteur d'air (f) comprennent des circuits d'intégration dans le temps des valeurs de puissance calorifique calculées pour délivrer en sortie des valeurs de quantité d'énergie.

**Claims**

1. Measuring apparatus for measuring the calorific power conveyed by a flow, in particular a gas flow, of fuel substance flowing along a main duct (1) for transit transport or distribution of a gaseous flow of fuel substance towards a downstream utilization zone at variable pressure, comprising a combustion chamber (110) specific to the measuring apparatus for burning a small known fraction ($q_{gas}$) of the mass flow rate ($Q_{gas}$) of the fuel substance flowing along the duct (1), and a system (104, 104') for measuring the air factor (f) applied to said combustion chamber (110) or for regulating the combustion in said combustion chamber (110) in order to maintain a constant air factor (f), characterized in that it comprises a main nozzle (20) having a sonic throat of variable cross-section which is disposed in the main duct (1), and a branch circuit (101) for taking a small known fraction ($q_{gas}$) of the mass flow rate ($Q_{gas}$) of the gaseous flow flowing along the main duct (1) in order to apply said small fraction ($q_{gas}$) to said controlled combustion chamber (110), in that the branch circuit (101) lies in a zone (8) of the main duct (1) situated upstream from the main nozzle (20) and comprises a secondary nozzle (120) whose sonic throat is of fixed geometry, in that the combustion chamber (110) of the branch circuit is fed with oxidizing air via a pipe (103) in which a meter device (106) is mounted to measure the mass flow rate ($q_{air}$) of the oxidizing air used for combustion of the fraction ($q_{gas}$) and applied to said combustion chamber (110) and in that it comprises computing and display or recording means (160) connected to said meter device (106) and to said system (104, 104') for measuring or regulating the air factor (f) in order to determine and display or record the value of the calorific power (Pu) of the substance flowing along said main duct (1) on the basis of data concerning the mass flow rates ($q_{air}$) of the oxidizing air delivered by said meter device (106) and data concerning the air factor (f) delivered by said measuring or regulation system (104, 104').

2. Apparatus according to claim 1, characterized in that the main nozzle (20) with a sonic throat and providing a downstream pressure regulation function has a throat whose flow section is variable and defined by the position of a moving cone (22) disposed inside the body of the nozzle (22) and moveable in translation along the axis of the nozzle relative to said throat, and in that a position sensor (24) is used to detect the displacement and the position of the moving cone (22) in order to provide the computing and display means (160) with data concerning the longitudinal position of said cone (22).

3. Apparatus according to any one of claims 1 and 2, characterized in that the branch circuit (101) has portions of reduced cross-section giving rise to localized head losses (zone Z2 or Z3).

4. Apparatus according to any one of claims 1 and 2, characterized in that the main duct (1) is provided with a filter (9) disposed upstream from the zone (8) for connecting the branch circuit (101) to the main duct (1).

5. Apparatus according to any one of claims 1 and 2, characterized in that the branch circuit (101) is provided with a second filter (109) having no significant head losses and disposed upstream from the zone (Z3) in which head losses are localized.

6. Apparatus according to any one of claims 1 and 2, characterized in that the branch circuit (101) is fitted with a through passage tap (81) disposed at the inlet to said circuit (101).

7. Apparatus according to any one of claims 1 and 2, characterized in that the controlled combustion chamber (110) is of the catalytic combustion type.

8. Apparatus according to any one of claims 1 to 7, characterized in that the meter device (106) for measuring the air mass flow rate comprises a low pressure positive displacement pump.

9. Apparatus according to any one of claims 1 to 8, characterized in that it includes a device for detecting variations in the combustion air factor, said device comprising a threshold probe for measuring the residual oxygen in the combustion products.

10. Apparatus according to any one of claims 1 to 9, characterized in that the meter device (106) for measuring the oxidizing air mass flow rate includes means for measuring volume, temperature, pressure, and humidity of the oxidizing air applied to the combustion chamber (110) specific to the apparatus.

11. Apparatus according to any one of claims 1 to 8, characterized in that the system (104') for measuring the air factor (f) includes means for analyzing the oxygen content of the combustion products coming from the combustion chamber (110) specific to the apparatus.

12. Apparatus according to claim 11, characterized in that the meter device (106) for measuring the oxidizing air mass flow rate also includes means for analyzing the oxygen content of the oxidizing air, and in that said means for analyzing the oxygen content are constituted by a single oxygen content analyzer applied in alternation to the ducts for exhausting the combustion products and to the duct (103) for supplying the oxidizing air.

13. Apparatus according to any one of claims 1 to 12, characterized in that the computing and display or recording means (160) connected to the meter device (106) and to the system (104, 104') for measuring or regulating the air factor (f) include circuits for integrating the calculated calorific power over time in order to deliver an output representative of a total quantity of energy.

**Patentansprüche**

1. Vorrichtung zum Messen der Wärmeleistung, die von einem insbesondere gasförmigen Strom eines energieerzeugenden Brennstoffs transportiert wird, welcher in einer Durchgangs-Transport- oder Verteiler-Hauptleitung (1) für einen gasförmigen Brennstoffstrom zu einer stromabwärtigen Verwendungszone mit variablem Druck zirkuliert, umfassend eine der Meßvorrichtung zugehörige Verbrennungskammer (110) zur Verbrennung einer bekannten geringen Fraktion ($q_{gaz}$) des Massendurchsatzes ($Q_{gaz}$) des in der Leitung (1) zirkulierenden Brennstoffs und eine Kette (104, 104') zur Messung des Luftfaktors (f), mit dem die Verbrennungskammer (110) beaufschlagt wird, oder zur Regelung der Verbrennung in der Verbrennungskammer (110) zwecks Aufrechthaltens eines konstanten Luftfaktors (f), dadurch gekennzeichnet, daß sie eine Hauptdüse (20) mit einer Schallverengung mit variablem Querschnitt, die in der Hauptleitung (1) angeordnet ist, und einen Nebenkreis (101) für eine bekannte geringe Fraktion ($q_{gaz}$) des Massendurchsatzes ($Q_{gaz}$) des in der Hauptleitung (1) zirkulierenden Gasstroms zur Beaufschlagung der geregelten Verbrennungskammer (110) mit dieser geringen Fraktion ($q_{gaz}$) aufweist, daß der Nebenkreis (101) in einer Zone (8) der Hauptleitung (1) beginnt, welche sich stromaufwärts der Hauptdüse (20) befindet, und eine Nebendüse (120) mit einer Schallverengung fixer Geometrie aufweist, daß die Verbrennungskammer (110) des Nebenkreises mit Verbrennungsluft über eine Rohrleitung (103) gespeist wird, an der eine Einrichtung (106) zur Zählung des Massendurchsatzes ($q_{air}$) der zur Verbrennung der Fraktion ($q_{gaz}$) verwendeten Verbrennungsluft, mit der die Verbrennungskammer (110) beaufschlagt wird, angeordnet ist, und daß sie Rechen- und Anzeige- oder Aufzeichnungsmittel (160) umfaßt, die mit der Zähleinrichtung (106) und der Kette (104, 104') zur Messung oder Regelung des Luftfaktors (f) zwecks Bestimmens und Anzeigens oder Aufzeichnens des Werts der Wärmeleistung (Pu) des in der Hauptleitung (1) zirkulierenden Brennstoffs, ausgehend von den Informationen über den Massendurchsatz ($q_{air}$) von Verbrennungsluft, die von der Zähleinrichtung (106) übermittelt werden, und über den Luftfaktor (f), die von der Meß- oder Regelungskette (104, 104') übermittelt werden, verbunden sind.

2. Vorrichtung nach Anspruch 1, dadurch gekennzeichnet, daß die Hauptdüse (20) mit Schallverengung, die die Funktion der Regelung des stromabwärtigen Drucks innehat, eine Verengung aufweist, deren variabler Durchtrittsquerschnitt durch die Position eines beweglichen Spitzkegels (22) definiert ist, welcher im Inneren des Düsenkörpers (20) angeordnet und entlang der Düsenachse in bezug auf die Verengung verschiebbar ist, und daß ein Positionsaufnehmer (24) zur Detektion der Verschiebung und der Position des beweglichen Spitzkegels (22) sowie zur Lieferung von Informationen betreffend die Längsposition dieses Spitzkegels (22) an die Rechen- und Anzeigemittel (160) verwendet wird.

3. Vorrichtung nach einem der Ansprüche 1 und 2, dadurch gekennzeichnet, daß der Nebenkreis (101) Abschnitte (Zone Z2 oder Z3) von reduziertem Querschnitt zur Lokalisierung von Druckverlusten aufweist.

4. Vorrichtung nach einem der Ansprüche 1 und 2, dadurch gekennzeichnet, daß die Hauptleitung (1) mit einem Filter (9) versehen ist, der stromaufwärts der Zone (8) der Verbindung des Nebenkreises (101) mit der Hauptleitung (1) vorgesehen ist.

5. Vorrichtung nach einem der Ansprüche 1 und 2, dadurch gekennzeichnet, daß der Nebenkreis (101) mit einem zweiten Filter (109) ohne bedeutende Druckverluste versehen ist, welcher stromaufwärts der Zone (Z3) zur Lokalisierung von Druckverlusten vorgesehen ist.

6. Vorrichtung nach einem der Ansprüche 1 und 2, dadurch gekennzeichnet, daß der Nebenkreis (101) mit einem Einweghahn (81) ausgestattet ist, der am Eingang dieses Kreises (101) angeordnet ist.

7. Vorrichtung nach einem der Ansprüche 1 und 2, dadurch gekennzeichnet, daß die geregelte Verbrennungskammer (110) eine katalytische Verbrennungskammer ist.

8. Vorrichtung nach einem der Ansprüche 1 bis 7, dadurch gekennzeichnet, daß die Einrichtung (106) zur Zählung des Massendurchsatzes der Verbrennungsluft eine Niederdruck-Verdrängerpumpe aufweist.

9. Vorrichtung nach einem der Ansprüche 1 bis 8, dadurch gekennzeichnet, daß sie eine Einrichtung zur Detektion von Veränderungen des Verbrennungsluftfaktors mit einer Schwellensonde zur Messung des Restsauerstoffs in den Verbrennungsprodukten aufweist.

10. Vorrichtung nach einem der Ansprüche 1 bis 9, dadurch gekennzeichnet, daß die Einrichtung (106) zur Zählung des Massendurchsatzes der Verbrennungsluft Mittel zur Messung des Volumens, der Temperatur, des Drucks und der Feuchtigkeit der Verbrennungsluft, mit welcher die der Vorrichtung zugehörige Verbrennungskammer (110) beaufschlagt wird, umfaßt.

11. Vorrichtung nach einem der Ansprüche 1 bis 8, dadurch gekennzeichnet, daß die Kette (104') zur Messung des Luftfaktors (f) Mittel zur Bestimmung des Sauerstoffgehalts der aus der der Vorrichtung zugehörigen Verbrennungskammer (110) austretenden Verbrennungsprodukte umfaßt.

12. Vorrichtung nach Anspruch 11, dadurch gekennzeichnet, daß die Einrichtung (106) zur Zählung des Massendurchsatzes der Verbrennungsluft auch Mittel zur Bestimmung des Sauerstoffgehalts der Verbrennungsluft aufweist, und daß die Mittel zur Bestimmung des Sauerstoffgehalts aus einem einzigen Sauerstoffgehaltanalysator bestehen, der abwechselnd an die Verbrennungsprodukte-Abführleitungen und an die Rohrleitung (103) zur Speisung mit Verbrennungsluft angelegt wird.

13. Vorrichtung nach einem der Ansprüche 1 bis 12, dadurch gekennzeichnet, daß die Rechen- und Anzeige- oder Aufzeichnungsmittel (160), die mit der Zähleinrichtung (106) und der Meß- oder Regelkette (104, 104') für den Luftfaktor (f) verbunden sind, Schaltungen zur zeitmäßigen Integration der errechneten Werte der Wärmeleistung zwecks Abgabe von Energiemenge-Werten am Ausgang umfassen.

$$Q_d = (K-1) \cdot q\,gaz$$

$$q\,gaz = \frac{Q\,gaz}{K}$$